# EUROPEAN PATENT APPLICATION

(11) **EP 3 527 206 A2**
(43) Date of publication of application: **21.08.2019**
(21) Application number: 17860633.1
(22) Date of filing: 06.12.2017
(51) Int. Cl.: A61K 31/231, A23L 33/10

(54) **COMPOSITION FOR PREVENTING OR TREATING HEPATITIS CONTAINING MONOACETYL DIACYLGLYCEROL COMPOUND**

(30) Priority: 13.10.2016 KR 20160132598
(71) Applicant: Enzychem Lifesciences Corporation, Daejon 34051 (KR)
(72) Inventor: SOHN, Ki Young, Seoul 07264 (KR); YOON, Sun Young, Daejeon 34199 (KR)
(74) Representative: Algemeen Octrooi- en Merkenbureau B.V.
(86) International application number: PCT/KR2017/014242
(87) International publication number: WO 2018/070854

(57) **Abstract**

Disclosed is a composition for preventing or treating hepatitis containing a monoacetyl diacylglycerol compound which not only can effectively prevent and treat hepatitis, but also is safe without any side effects when used. The composition contains a monoacetyl diacylglycerol compound represented by Chemical Formula 1 in the specification as an active ingredient. In Chemical Formula 1 of the specification, R₁ and R₂ are each independently a fatty acid group having 14 to 22 carbon atoms.

## Description

### [TECHNICAL FIELD]

The present invention relates to a composition for preventing or treating hepatitis containing a monoacetyl diacylglycerol compound. More specifically, the present invention relates to a composition for preventing or treating hepatitis containing a monoacetyl diacylglycerol compound which not only can effectively prevent and treat hepatitis, but also is safe without any side effects when used.

### [BACKGROUND ART]

The liver is one of the most important metabolic organs in the body, and performs important functions such as bile secretion, nutrient storage, and detoxification. Thus, when an abnormality occurs in the liver, metabolic disorders such as carbohydrate metabolism, lipid metabolism, protein and nitrogen metabolism, amino acid metabolism, protein metabolism and liver brain disease, vitamin metabolism, malabsorption and the like occur, and liver diseases can be exacerbated by infections, fatty liver, liver cirrhosis and the like. Among liver diseases known to date, disorders exhibiting various hepatic dysfunctions such as acute hepatitis caused by drugs, etc., fatty liver caused by drinking, etc., acute hepatitis caused by virus infection, chronic hepatitis caused by transformation of electric acute hepatitis or liver cirrhosis accompanied by electric disorder are known.

Among them, hepatitis is a disease caused by T cell-mediated immune response and hepatic inflammation. Under these conditions, the increase in new white blood cells and the site of these cells in the liver induce the onset of diseases. The pathogenesis of hepatitis is widely known, but the treatment goals (subject, target) are still not satisfied. Concanavalin A (below, ConA) induces T cell activation and T cell-mediated liver injury (Gantner F, Leist M, Lohse AW, Germann PG, Tiegs G. Concanavalin A-induced T-cell- mediated liver injury in mice: the role of tumor necrosis factor. Hepatology 1995; 21:190-198). T cells and macrophages secrete various cytokines such as IFNγ, TNFα, and IL-4 in the hepatocyte-damaged state, and in particular, IL-4 and IL-13 activate STAT6, which are a major cause of Th2 differentiation, tissue attachment, and various effects of inflammation and hematopoietic tissues. IL-4 produces IL-8 by human umbilical vein endothelium (Striz I, Mio T, Adachi Y, Robbins RA, Romberger DJ, Rennard SI. IL-4 and IL-13 stimulate human bronchial epithelial cells to release IL-8. Inflammation 1999; 23:545-555), and induces regulation of neutrophil trafficking through the STAT3 pathway of this cytokine. Further, according to a recent report, STAT6 activity by IL-4 may induce the deterioration of liver injury by promoting the migration of leukocytes (Jaruga B, Hong F, Sun R, Radaeva S, Gao B. Crucial role of IL-4/STAT6 in T cell-mediated hepatitis: up-regulating eotaxins and IL-5 and recruiting leukocytes. J Immunol 2003; 171:3233-3244).

In addition, efforts have been made to develop drugs for treating such hepatic diseases, but to date only a few drugs have been clinically effective for treating liver diseases. As the most useful method for search for the pharmacological effect of these drugs, methods for measuring the protective effect against a rapid increase in the level of GOT and GPT in serum induced by carbon tetrachloride (CCl₄) are known. It has been known that drugs searched as substances capable of inhibiting hepatitis caused by carbon tetrachloride exhibit liver-protective actions and detoxifying effects (see, Kiso, Y., Shoyaku, 36:238-243, 1982; Glende, E. A., Biochem. Pharmacol., 25:2163-2169, 1976). Further, galactosamine, which is known as a substance capable of inducing hepatic diseases similarly to carbon tetrachloride, can specifically act on the liver and causes various diseases. It mainly induces hepatocyte necrosis and inflammation in hepatocyte and hepatic portal vein, and when induced to chronic hepatic disease, it is known that it can induce cirrhosis and cellular tumors.

To date, various products have been developed as therapeutic agents for hepatic diseases, but the effectiveness has been substantially demonstrated in only a few types such as silymarin (SIL), biphenyl dimethyl dicarboxylate (DDB) and ursodeoxycholic acid (UDCA). These therapeutic agents have a disadvantage in that the types of hepatic diseases that can be applied are limited. This is because, due to the various functions of the liver, when administering a therapeutic agent to treat one type of disease, desired diseases may be treated, but unexpected side effects may occur, and when hepatic diseases are misdiagnosed, the hepatic disease may be aggravated by the inappropriate administration of the therapeutic agent. Furthermore, the drugs developed to date are mainly drugs for treating hepatic diseases that have developed, and the research results of drugs that can prevent hepatic diseases in advance are deficient. Accordingly, there is a pressing need to develop a drug which can be used for the prevention and treatment of hepatic diseases without side effects, and thus, efforts have been made to extract components showing the preventive and treatment of hepatic diseases without side effects from natural product extracts. For example, it has been reported that the extract extracted from green tea may increase the activity of UDP-glucuronosyl transferase to increase the release of carcinogens in the liver (see, A. Bu-Abbas et al., Food and Chemical Toxicology, 33(1):27-30, 1995; O. S. Sohn et al., Food and Chemical Toxicology, 36:617-621, 1998). However, it did not demonstrate substantial therapeutic effects of liver cancer, and there is no progress in research on extracts that can improve other hepatic diseases.

Therefore, there has always been a need to develop a composition that has no side effects and can be used effectively for the prevention and treatment of hepatitis.

### [DETAILED DESCRIPTION OF THE INVENTION]

### [Technical Problem]

Accordingly, it is one object of the present invention to provide a composition for preventing or treating hepatitis containing a monoacetyl diacylglycerol compound which can effectively prevent or treat hepatitis without side effects.

### [Technical Solution]

In order to achieve the object above, one aspect of the present disclosure provides a pharmaceutical composition for preventing or treating hepatitis containing a monoacetyl diacylglycerol compound represented by the following Chemical Formula 1 as an active ingredient: 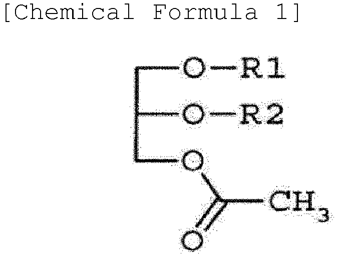 in Chemical Formula 1, R₁ and R₂ are each independently a fatty acid group having 14 to 22 carbon atoms.

Another aspect of the present disclosure provides a health functional food composition which contains the aforementioned composition and is capable of preventing or improving hepatitis.

Still another aspect of the present disclosure provides a method for preventing or treating hepatitis containing administering the aforementioned composition to a subject suspected of hepatitis.

### [ADVANTAGEOUS EFFECTS]

The composition according to the present invention is not only effective in preventing and treating hepatitis, but also can be safely used without side effects.

### [BRIEF DESCRIPTION OF THE DRAWINGS]

FIG. 1 is a diagram showing an example of a mechanism for preventing hepatitis using PLAG of the present invention.
FIG. 2 is a graph showing changes in cytokines in plasma when the composition of the present invention is administered.
FIG. 3 is a graph (A) and RT-PCR photograph (B) showing changes of cytokine in liver tissue when the composition of the present invention is administered.
FIG. 4 is a graph showing changes in neutrophils and white blood cells in blood (A) and liver tissue (B) when the composition of the present invention is administered.
FIG. 5 is a photograph showing whether neutrophil cells located at the site of injury in liver tissue remain when the composition of the present invention is administered.
FIG. 6 shows the relaxation effect of PLAG on liver injury when the composition of the present invention is administered.
FIG. 7 is a graph showing the results of WST assay of HepG3B cells and HL-60 cells of liver cell origin depending on the dose of PLAG when the composition of the present invention is administered.
FIG. 8 is a figure showing the inhibitory effect of IL-4-induced STAT6 activity and related signaling mechanism depending on the dose of PLAG when the composition of the present invention is administered.
FIG. 9 is a figure showing the structure (A) and the effect comparison (B and C) of PLAG and PLG of the present invention.
FIG. 10 is a graph showing the transcriptional activity of STAT6 when the composition of the present invention is administered.
FIG. 11 is a figure showing the change in expression of IL-8 and VCAM-1 and the effect of decreasing the cell adhesion ability depending on the dose of PLAG when the composition of the present invention is administered.

### [DETAILED DESCRIPTION OF THE EMBODIMENTS]

Hereinafter, the present invention will be described in more detail with reference to the accompanying drawings.

The pharmaceutical composition for preventing or treating hepatitis contains a monoacetyl diacylglycerol compound represented by the following Chemical Formula 1 as an active ingredient: 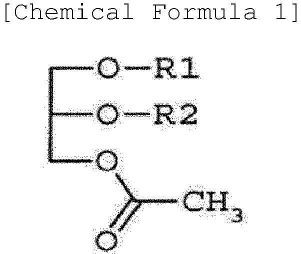 in Chemical Formula 1, R₁ and R₂ are each independently a fatty acid group having 14 to 22 carbon atoms, preferably a fatty acid group having 15 to 20 carbon atoms.

As used herein, the term "monoacetyl diacylglycerol compound" refers to a glycerol derivative having one acetyl group and two acyl groups, and is also simply referred to as monoacetyl diacylglycerol (MADG). In Chemical Formula 1, R₁ and R₂ are each independently a fatty acid group having 14 to 22 carbon atoms (in fatty acids, it refers to the remaining part from which the hydroxy group is excluded), and may be preferably palmitoyl, oleoyl, linoleoyl, linolenoyl, stearoyl, myristoyl and arachidonoyl, but is not limited thereto. More preferably, the combination of R₁ and R₂ may include oleoyl/palmitoyl, palmitoyl/oleoyl, palmitoyl/linoleoyl, palmitoyl/linolenoyl, palmitoyl/arachidonoyl, palmitoyl/stearoyl, palmitoyl/palmitoyl, oleoyl/stearoyl, linoleoyl/palmitoyl, linoleoyl/stearoyl, stearoyl/linoleoyl, stearoyl/oleoyl, myristoyl/linoleoyl, or myristoyl/oleoyl, and most preferably, the combination of R₁ and R₂ may be palmitoyl/ linoleoyl. Further, in terms of optical activity, the monoacetyl diacylglycerol compound may be (R)-form, (S)-form or a racemic mixture, and may be preferably a racemic mixture. Also, all stereoisomers of the above-mentioned compound are within the scope of the present invention.

For example, the monoacetyl diacylglycerol compound may be a compound represented by the following Chemical Formula 2:

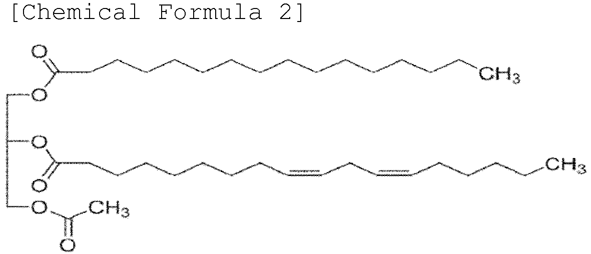

The compound represented by Chemical Formula 2 is 1-palmitoyl-2-linoleoyl-3-acetyl-rac-glycerol, which corresponds to the compound where R₁ and R₂ of Chemical Formula 1 are palmitoyl and linoleoyl, respectively. It is also named as "PLAG" or "EC-18" as needed.

The monoacetyl diacylglycerol compound of the present invention may be extracted/separated from deer antlers or may be produced by known organic synthesis methods. For example, a deer antler is extracted with hexane, and extracting the extraction residue with chloroform, followed by subjecting the thus-obtained extraction liquid to distillation under reduced pressure to obtain chloroform extracts of the deer antler. The hexane and chloroform as the solvents used for the extraction are used in an amount sufficient to immerse the deer antler. In general, about 4 to 5 liters of each of hexane and chloroform is used for 1 kg of deer antler, but the type of extraction solvents and amount thereof are not limited thereto. The chloroform extracts of the deer antler obtained by this method is further fractionated and purified by silica gel column chromatography or TLC to obtain the monoacetyl diacylglycerol compound used in the present invention. As an eluent used in the chromatography purification step, chloroform/methanol, hexane/ethyl acetate, and hexane/ethyl acetate/acetic acid may be used, but is not limited thereto.

One example of the method for chemically synthesizing the monoacetyl diacylglycerol compound is disclosed in Korean Patent No. 10-0789323. According to the method of this patent, the desired monoacetyl diacylglycerol compound can be synthesized by the steps of: (a) preparing 1-R₁-3-protecting group-glycerol by adding a protecting group at the position 3 of 1-R₁-glycerol; (b) preparing 1-R₁-2-R₂-3-protecting group-glycerol by introducing R₂ group at the position 2 of 1-R₁-3-protecting group-glycerol; and (c) performing a deprotection reaction and acetylation reaction of 1-R₁-2-R₂-3-protecting group-glycerol at the same time. Alternatively, acetolysis of phosphatidylcholine may be used, but is not limited thereto.

The monoacetyl diacylglycerol compound exhibits excellent activities in improving, preventing or treating hepatitis.

The content of the monoacetyl diacylglycerol compound contained in the pharmaceutical composition of the present invention is not particularly limited, but is preferably contained in an amount of 0.0001 to 100.0% by weight, preferably 0.001 to 99% by weight, more preferably 0.001 to 50% by weight, most preferably 0.01 to 20% by weight, based on the total weight of the composition. If desired, the pharmaceutical composition of the present invention may further include other active ingredients having a therapeutic effect of hepatitis. For example, when prepared into a soft capsule, it may contain, as an antioxidant, from 0 to 5% by weight, preferably from 0.1 to 3% by weight, more preferably from 0.5 to 1.5% by weight of alpha-tocopherol, and the like. The pharmaceutical composition may have a formulation such as tablets, pills, powders, granules, capsules, suspensions, solutions, emulsions, syrups, sterile aqueous solutions, non-aqueous solvents, suspensions, freeze-dried preparations, suppositories, etc., and may be variously formulated for oral or parenteral administration. The composition of the present invention may be formulated with commonly used diluents or excipients, such as fillers, extenders, binders, wetting agents, disintegrants, surfactants, *etc.* Therefore, the pharmaceutical composition of the present invention may further include carriers, excipients, diluents, etc., which are conventionally used in the production of pharmaceutical compositions. Solid formulations for oral administration include tablets, pills, powders, granules, capsules, *etc.,* and these solid formulations are prepared by mixing one or more active compounds with at least one excipient, for example, starch, calcium carbonate, sucrose, lactose, gelatin, etc. In addition to simple excipients, lubricants such as magnesium stearate, talc, etc. may also be used. Liquid formulations for oral administration include suspensions, solutions, emulsions, syrups, etc., and may contain various excipients, for example, wetting agents, flavoring agents, aromatics and preservatives, in addition to water and liquid paraffin, which are frequently used-simple diluents. Formulations for parenteral administration include sterilized aqueous solutions, non-aqueous solutions, suspensions, emulsions, freeze-dried preparations, suppositories, etc. As non-aqueous solvents or suspending agents, propylene glycol, polyethylene glycol, vegetable oils such as olive oil, injectable esters such as ethyl oleate, etc. can be used. As the base of the suppositories, witepsol, Macrogol, Tween 61, cacao butter, laurin fat, glycerogelatin, etc. can be used.

The compound of the present invention may be administered in a pharmaceutically effective amount. As used herein, the term "pharmaceutically effective amount" refers to an amount sufficient to treat diseases, at a reasonable benefit/risk ratio applicable to any medical treatment. The effective dosage level of the composition may be determined depending on the subject's type, age, sex, weight, the type of a disease, the severity of a disease, the activity of a drug, the sensitivity to a drug, the form of a drug, the route of administration, the duration of administration, the excretion rate, drugs used in combination with the composition, and other factors known in the medical field. The composition of the present invention may be administered as an individual therapeutic agent or in combination with other therapeutic agents, and may be administered sequentially or simultaneously with other therapeutic agents. It is important to administer the composition in the minimum amount that can exhibit the maximum effect without causing side effects, in consideration of the above-described factors, and this amount can be easily determined by a person skilled in the art. The preferred dose of the compound of the present invention may be determined by one who performs treatments within the scope of appropriate medical decisions, and may be typically administered at a dose of 0.001 to 2,000 mg/kg, preferably 0.05 to 400 mg/kg, and more preferably 0.1 to 200 mg/kg once or in a few divided doses in a day. For example, the compound may be administered once or several times at a dose of 1 mg to 1 g, preferably 30 mg to 120 mg per day for an adult with a body weight of 60 kg. Since the compound of the present invention has no toxicity and side effects, it can be administered for a long period of time for the purpose of prevention or treatment. The composition of the present invention may be applied to any subject as long as the purpose is to prevent or treat hepatitis. Examples of the subject include human, non-human animals such as monkeys, dogs, cats, rabbits, marmots, rats, mice, cows, sheep, pigs, and goats, etc., and mammals.

Further, the present invention provides a health functional food composition which contains a monoacetyl diacylglycerol compound represented by the following Chemical Formula 1, as an active ingredient, and is capable of preventing or improving hepatitis: 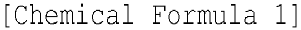 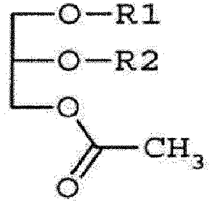 in Chemical Formula 1, R₁ and R₂ are each independently a fatty acid group having 14 to 22 carbon atoms.

That is, by incorporating the monoacetyl diacylglycerol compound of the present invention into the health functional food composition, hepatitis of the subject may be prevented or improved. The monoacetyl diacylglycerol compound and hepatitis are as described above. When the compound of the present invention is incorporated into the health functional food composition, the mixed amount of the active ingredients may be appropriately determined depending on the intended use. Generally, during the production of food or beverage, the compound of the present invention is added in an amount of preferably 0.01 to 15 parts by weight, more preferably 0.02 to 10 parts by weight, and still more preferably 0.3 to 1 part by weight, based on 100 parts by weight of the raw material. However, in the case of long-term intake for the purpose of health control and hygiene, the amount may be less than or equal to the above range, and may be used in an amount exceeding the above range, if necessary. The type of the health functional food capable of containing the compound of the present invention is not particularly limited, but specifically, the health functional food may include meats, sausages, bread, chocolates, candies, snacks, confectionery, pizza, instant noodles, other noodles, gums, daily products including ice creams, various soups, beverages, teas, drinks, alcoholic beverages, and vitamin complexes, and may also include any conventional health functional food and animal feeds.

In addition, when the health functional food composition is used in the form of a beverage, it may contain conventional sweeteners, flavoring agents, natural carbohydrates, etc., as additional ingredients. Examples of the natural carbohydrates include monosaccharides such as glucose and fructose, disaccharides such as maltose and sucrose, polysaccharides such as dextrin and cyclodextrin, and sugar alcohols such as xylitol, sorbitol and erythritol. The ratio of the natural carbohydrates may preferably be about 0.01 to 0.04 g, more preferably 0.02 to 0.03 g per 100 mL of the composition of the present invention. Examples of the sweeteners include natural sweeteners such as thaumatin and stevia extracts, and artificial sweeteners such as saccharin and aspartame. In addition to the above, the health functional food composition of the present invention may contain various nutrients, vitamins, electrolytes, flavoring agents, colorants, pectic acid and salts thereof, alginic acid and salts thereof, organic acids, protective colloidal thickening agents, pH controlling agents, stabilizing agents, preservatives, glycerin, alcohol, carbonizing agents used in carbonated beverages, etc. Moreover, the health functional food composition of the present invention may contain fruits as used in preparing natural fruit juices, fruit juice beverages and vegetable beverages.

In addition, the present invention provides a method for preventing or treating hepatitis including:
administering the pharmaceutical composition to a subject suspected of hepatitis. That is, by administering the compound of the present invention to subject suspected of hepatitis, hepatitis can be efficiently prevented or treated. As used herein, the subject suspected of hepatitis refers to a subject who has hepatitis or has the potential of having hepatitis. In the therapeutic method of the present invention, the type of monoacetyl diacylglycerol compound, the dose of monoacetyl diacylglycerol compound, and hepatitis are as described above. As used herein, the term "administration" refers to introducing the pharmaceutical composition of the present invention to a subject suspected of hepatitis via any appropriate method. The administration route may include various oral or parenteral routes as long as the composition can reach a desired tissue, and for example, oral administration, intraperitoneal administration,
transdermal administration (topical application, etc.), intravenous administration, intramuscular administration, subcutaneous administration, intradermal administration, intranasal administration, intrarectal administration, intraperitoneal administration, etc. may be used, but is not limited thereto. As used herein, the term "prevention" refers to any activity that inhibits or delays occurrence of hepatitis by administering the composition of the present invention. As used herein, the term "treatment" refers to any activity that alleviates or positively changes symptoms of hepatitis by the composition of the present invention. As used herein, the term "improvement" refers to any activity that alleviates or ameliorates symptoms of subjects suspected of or having hepatitis using the composition of the present invention.

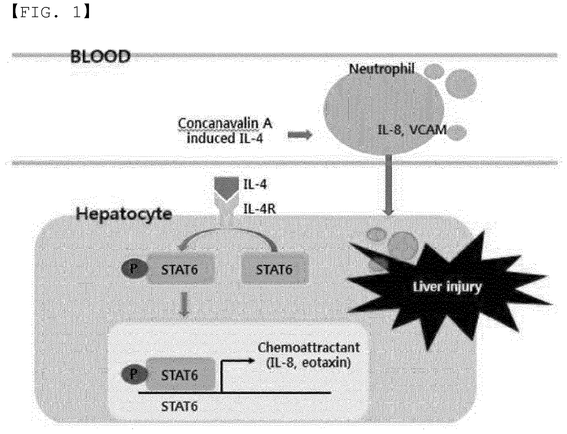

FIG. 1 is a diagram showing an example of a mechanism for preventing hepatitis using PLAG of the present invention. As shown in FIG. 1, IL-4 is induced by concanavalin A, and the induced IL-4 induces phosphorylation of STAT6 while migrating neutrophils from the blood to the liver, whereby neutrophils are recruited, and phosphorylated STAT6 secretes chemokines such as IL-8, iotaxin and the like, which in turn promotes migration of neutrophil cells to induce damage to liver tissue. Administration of the composition of the present invention by the above mechanism suppresses the expression and/or activity of IL-4, IL-6, MIP-2 (e.g., CXCL2 and/or IL-8, etc.), and blocks phosphorylation of PKC-ζ, PKCδ, PKCθ, STAT6 and mixtures thereof, expression of adsorbed molecules and/or excessive migration from blood to the liver, thereby preventing or treating hepatitis.

Hereinafter, the present invention will be described in more detail by way of Examples. However, the present invention is not limited to or by the Examples.

In order to confirm the preventive and therapeutic effects of hepatitis by 1-palmitoyl-2-linoleoyl-3-acetylglycerol (EC-18 or PLAG), the experiment was performed using an attenuation model of hepatitis induced by concanavalin A (hereinafter, referred to as ConA). As an experimental model, 10-week-old Balb/c mice (Koatech, Korea) were maintained under specific pathogen free (SPF) environment and used, and PLAG and PLG (ENZYCHEM Lifesciences, Korea) were prepared before or after *in vivo* or *in vitro* treatment. In addition, Hep3B, HepG2, EL-4 and HL-60 cell lines (American Type Culture Collection, ATCC) were incubated under 5% CO₂ and a temperature of 37°C. The values in the comparison between groups performed *in vivo* were analyzed using one analysis method of the analysis of variance (ANOVA), and the values in the comparison of two experimental groups performed *in vitro* were analyzed using independent student's t test. Statistical significance was assumed to be p <0.05.

### [Example 1] Preparation of Control Group and Experimental Group

Mice were randomly divided into three groups: control group, Con A-treated group, and PLAG-pretreated group. The Con A and PLAG were all dissolved in PBS (phosphate buffer saline) and used. PBS or PLAG was orally administered to the mice, and after 2 hours, 20 mg/kg BW of Con A was intravenously injected into each of the Con A-treated group and the PLAG-pretreated group. Thereafter, PLAG was orally administered to mice at a dose of 100 mg/kg BW. Here, BW means body weight.

### [Experimental Example 1] Plasma and tissue cytokine analysis

Blood samples were obtained from cardiac punctures 18 hours after administration of Con A in the mice of Example 1 and centrifuged at 3,000 rpm for 10 minutes. For the measurement of cytokines in liver tissue ligate liquids, the animals were sacrificed and the liver was washed with PBS. The liver was homogenized and filtered using a 70 µm mesh cell strainer (BD Falcon cell strainer, BD Biosciences, Bedford, MA) in PBS. The supernatant was also collected by centrifugation at a rate of 2,000 rpm for 5 minutes. The lower cell pellet was resuspended in PBS and analyzed for complete blood cell count (CBC). Plasma samples and supernatants of liver lysate were maintained at minus 20°C until cytokines were measured. The concentrations of IL-6, IL-10, IL-4, MIP-2 and IFNγ were detected using Set ELISA.

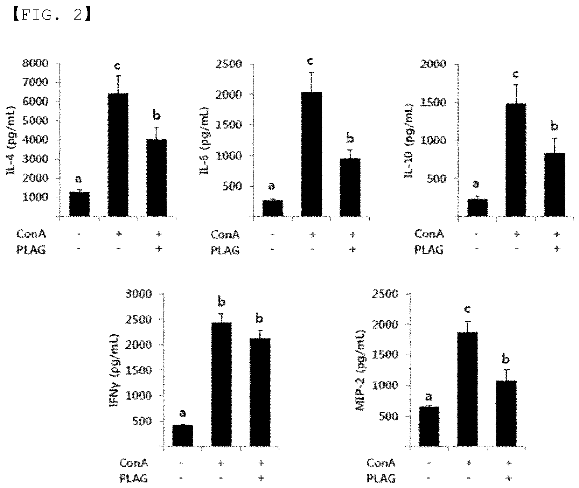

FIG. 2 is a graph showing changes in cytokines in plasma when the composition of the present invention was administered. As shown in FIG. 2, PLAG decreased IL-4, IL-6, IL-10 and MIP-2 in mouse plasma to which Con A was administered. Specifically, IL-4, IL-6, IL-10 and MIP-2 of mouse plasma injected with Con A were considerably higher than those of control rats, IL-4, IL-6, IL-10 and MIP-2, which were increased by ConA stimulation, considerably reduced in rats after intake of PLAG, and the concentration of IFNγ increased in the ConA-induced group was not significantly reduced compared to mice not treated with PLAG. Therefore, it can be seen that PLAG can reduce the secretion of cytokines that induce the onset associated with hepatitis induced by Con A treatment.

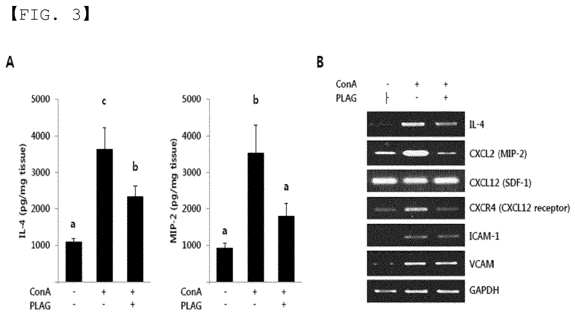

Further, FIG. 3 is a graph (A) and RT-PCR photograph (B) showing changes of cytokine in liver tissue when the composition of the present invention was administered. As shown in FIG. 3, high protein and mRNA levels of IL-4 and MIP-2 in the liver were observed in liver tissue of ConA-administered mice. Increased expression of the cytokines (IL-4, MIP-2, etc.) in the group to which ConA was administered significantly reduced in the group to which PLAG was further administered. These results show that PLAG regulates cytokines such as IL-4 and MIP-2 levels in plasma (see FIG. 3A). In addition, the regulation of chemokine receptor CXCR4 mRNA and the adsorbed molecules, ICAM-1 and VCAM was assessed using RT-PCR in ConA-induced liver tissue. As a result, it can be seen that PLAG down-regulates the expression of CXCR4 mRNA and does not affect the adsorbed molecules containing ICAM-1 and VCAM (see Fig. 3B).

### [Experimental Example 2] Hematological analysis of whole blood and liver samples

Whole blood and liver samples were collected from the mice of Example 1 18 hours after administration of Con A. The cleaved liver collected from the mouse was homogenized as described in Experimental Example 1. Complete blood count (CBC) analysis included whole white blood cell quantification, part of neutrophils and lymphocytes of whole white blood cells, and hematology analysis was performed using Mindray BC 5300 (Shenzhen Mindray Biomedical Electronics, China).

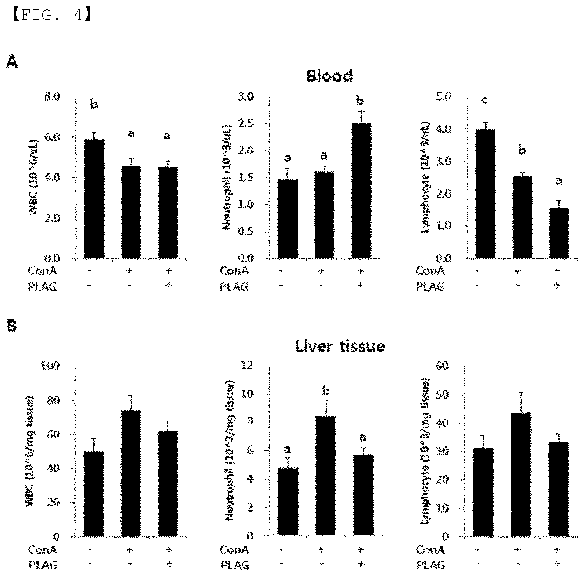

FIG. 4 is a graph showing changes in neutrophils and white blood cells in blood (A) and liver tissue (B) when the composition of the present invention was administered. As shown in FIG. 4, the number of circulating white blood cells was slightly down-regulated in ConA-stimulated mice, and circulating neutrophils were significantly increased in the group to which PLAG was additionally administered (Fig. 4A). In addition, the number of white blood cells present in the liver was increased in Con-A-treated mice, and the number of neutrophils present in the liver was significantly reduced in the group to which PLAG was additionally administered (FIG. 4B). These results show that migration of white blood cells from liver in the blood is accelerated in ConA-administered mice, and migration of neutrophil returns to its original state in the group to which PLAG was additionally administered.

### [Experimental Example 3] Histological analysis

The site of liver was collected from a mouse 8 hours after administration of Con A to the mouse of Example 1. The site of liver was fixed in 10% neutral buffered formalin, embedded in paraffin and cut to a thickness of 5 um. After removing the paraffin and rehydration, the flakes were stained with hematoxylin and eosin. In addition, for identification of neutrophils, the site of liver was stained with anti-mouse neutrophil antibody. All samples were evaluated histologically under a Leica (Leica, Wetzlar, Germany) optical microscope, and digital images were taken at X200 magnification.

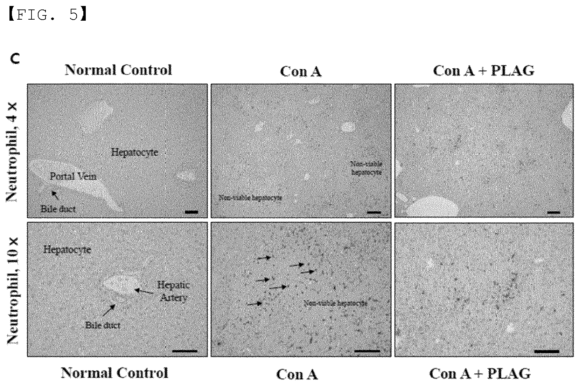

FIG. 5 is a photograph showing whether neutrophil cells located at the site of injury in liver tissue remained when the composition of the present invention was administered. As shown in FIG. 5, administration of ConA in the liver induces a large number of leukocyte infiltration, which may in turn affect autologous tissue destruction, but by administrating PLAG, it has the potential to alleviate neutrophil migration into the liver. It can be seen that total neutrophil counts were measured by immunohistological staining using anti-neutrophil antibodies, and liver tissue treated with ConA had severe liver cell damage, whereas the hepatocyte treated with PLAG was hardly damaged. Therefore, neutrophils enhanced by stimulation with ConA can be effectively alleviated by performing PLAG treatment. In FIG. 5, the scale bar means 200 um.

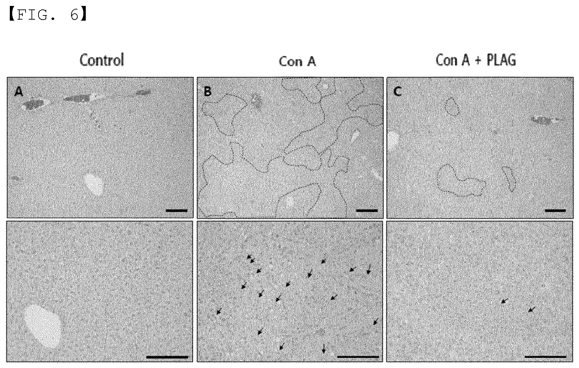

FIG. 6 shows the relaxation effect of PLAG on liver injury when the composition of the present invention was administered. As shown in FIG. 6, the relaxation effect of PLAG on liver injury was tested using hematoxylin-eosin staining. Histological examination of the liver site shows the consequences of intravenous administration of ConA such as severe hepatocyte damage within 8 hours, structure of the capillary sinusoid and neutrophil infiltration of liver. However, when PLAG was administered at 100 mg/kg, PLAG treatment markedly reduced the pathological changes as described above, including neutrophil infiltration with rare distribution and very few necrotic foci. Therefore, it can be seen that PLAG reduces ConA-induced hepatitis.

### [Experimental Example 4] Western blot assay

Hep3B cells were plated in 12 well plates (2.5x10⁵ cells / well) and treated with PLAG at various concentrations (0.1, 1, 10, 100 ug/mL), and after 2 hours, activated with IL-4 for 1 hour. Whole protein extracts were immunoblotted with phospho-STAT6, STAT6, phospho-JAK1 and phosphor-PKCξ/λ Abs (cell signaling).

HL-60 cells were plated in 12-well plates (1x10⁶/ well) and treated as described above. Total protein extracts were immunoblotted with phospho-PKC homologous proteins (δ, θ, ξ/λ, α/β) (Cell Signaling) and GAPDH Abs (Santa Cruz Biotechnology Inc., CA). Detection was performed using Immobilon Western Chemiluminescent HRP Substrate (Millipore Corporation, Billerica, Mass.).

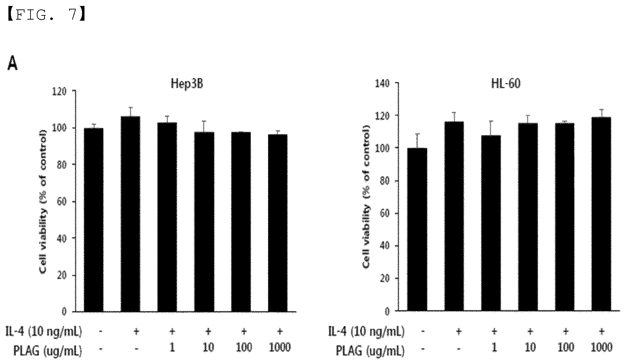

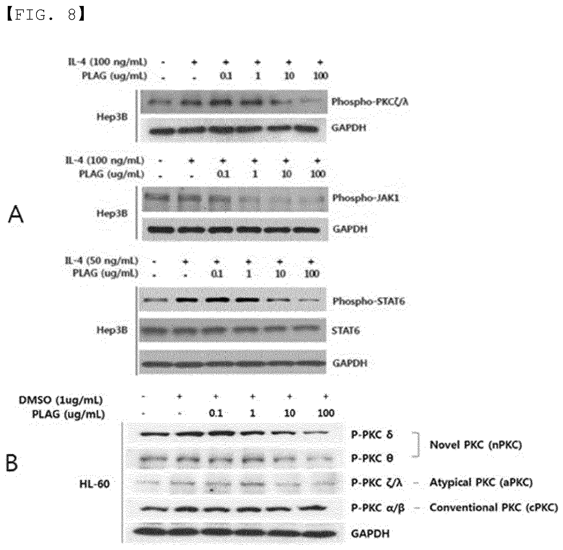

FIG. 7 is a graph showing the results of WST assay of HepG3B cells and HL-60 cells of liver cell origin depending on the dose of PLAG when the composition of the present invention was administered, and FIG. 8 is a graph showing the inhibitory effect of IL-4-induced STAT6 activity and related signaling mechanism depending on the dose of PLAG when the composition of the present invention was administered. As shown in FIGS. 7 and 8, the intracellular mechanism for reducing liver injury using PLAG was confirmed in vitro. HepG2 and Hep3B were used as liver cells, and HL-60 was used as neutrophil cells. Cytotoxicity of EC-18 in liver cells and leukocytes was confirmed by WST assay. Survival of Hep3B and HL-60 cells was not affected during 24 hours of incubation with PLAG each having a concentration of 1,000 ug/mL or greater (see FIG. 7). Based on the results of the WST assay, 100, 10, and 1 ug/mL of PLAG were used for the cells. The activities of PKC-ξ/λ, JAK1 and STAT6 played an important role in ConA-induced hepatitis, and these kinases were increased by IL-4. Western blot assay showed that phosphorylation of PKC and STAT6 was induced by IL-4 stimulation, and phosphorylation of these kinases (PKC, STAT6, etc.) was considerably alleviated by treating PLAG in a dose-dependent manner (see FIG. 8). Therefore, it can be seen that PLAG can effectively control IL-4-induced hepatitis by alleviating PKC-ξ/λ activity. Further, PLAG affects the relaxation of the activity of PKCδ and PKCθ in the PKC isotype, while it did not affect the relaxation of PKCα/β activity (see FIG. 8B) .

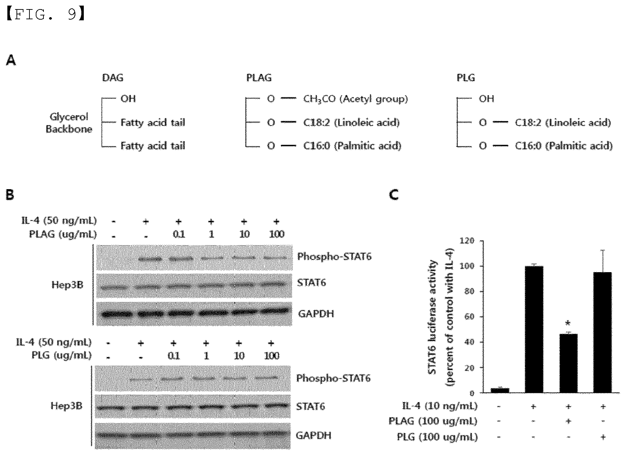

Further, FIG. 9 is a figure showing the structure (A) and the effect comparison (B and C) of PLAG and PLG of the present invention. As shown in FIG. 9, the selectivity of PLAG was confirmed by comparing PLAG and PLG in the respective STAT6 phosphorylation and transcription activities. PLG is a prototype of DAG, and PLAG is a type of acetylated DAG (see FIG. 9A). The specificity of PLAG function for alleviation of live injury was indirectly examined through STAT6 activity assay. Western blot assay using anti-phospho-SAT6 showed that phosphorylation of STAT6 was significantly reduced in PLAG treated Hep3B cells but not altered in PLG treated cells. The results as described above show that PLAG plays a very special role in protecting against liver injury in the hepatitis induced by ConA.

### [Experimental Example 5] RNA isolation and RT-PCR analysis

Total RNA was isolated from the liver homogenate with TRIzol reagent (Incitrogen, Carlsbad, CA). HL-60 cells were plated in 12-well plates (1x10⁶ / well) and treated in the same manner as in Experimental Example 4. cDNA was synthesized from total cellular RNA using M-MLV reverse transcriptase and oligo-dT (Promega, Madison, Wis.). PCR primers (Bioneer, Daejeon) were designed using Primer 3 program, and mouse and human primer sequences are shown in Table 1 below.

**[Table 1]**

| Name | Forward primer (5'-3') | Reverse primer (5'-3') |
|---|---|---|
| mIL-4 | TGACGGCACAGAGCTATTGA | TGTTCTTCGTTGCTGTGAGG |
| mCXCL2 (MIP-2) | AGTGAACTGCGCTGTCAATG | CTTTGGTTCTTCCGTTGA GG |
| mCXCL12 (SDF-1) | GAGCCAACGTCAAGCATCTG | CGGGTCAATGCACACTTG TC |
| mCXCR4 (CXCL12 receptor) | GGGGACATCAGTCAGG | GTGGAAGAAGGCGAGGG |
| mVCAM | CCTCACTTGCAGCACTACGGGCT | TTTTCCAATATCCTCAATGACGGG |
| hIL-8 | GCAGGAATTCACCTCAAGA | CTTCAGGAACAGCCACCAAT |
| hVCAM | AGTTGAAGGATGCGGGAGTA | TCTCCAGTTGAACATATCAAGCA |

The amount of cDNA amplification of the reaction samples was confirmed by 2% agarose gel (Roche Applied Science, Salt Lake City, UT) electrophoresis.

### [Experimental Example 6] Luciferase reporter gene analysis

HepG2 and Hep3B cells were transfected into reporter luciferase plasmids containing four pairs of STAT6 binding sites (p4xSTAT6-Luc2P; Addgene, Cambridge, MA), and the reporter vector was transfected into the Attractene reagent and cell line. For the activity of STAT6 reporter vectors, 10 ng/ml of IL-4 was treated with cells for 6 hours, and the luciferase activity was measured by a luminometer using a luciferase assay system. The luciferase activity of each sample was normalized to those corresponding to the PLAG transduced samples.

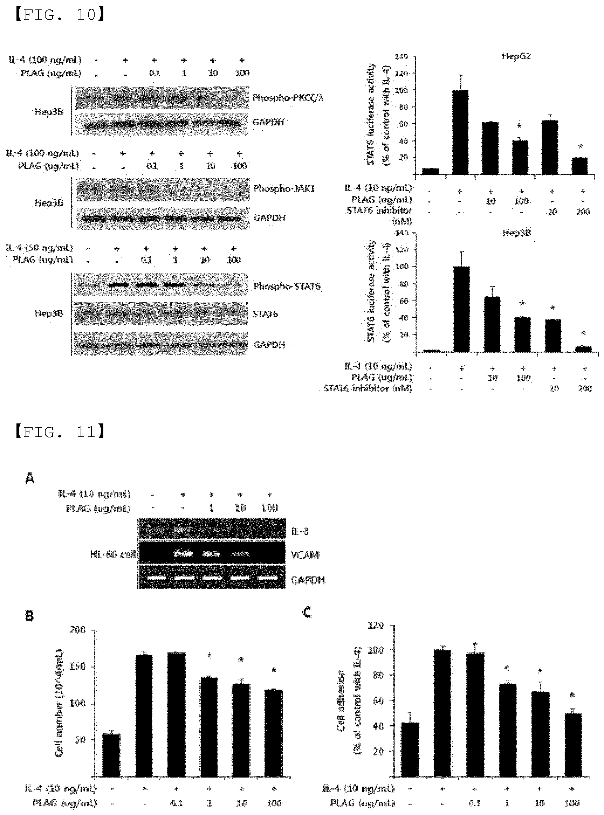

FIG. 10 is a graph showing the transcriptional activity of STAT6 when the composition of the present invention was administered. As shown in FIG. 10, it can be seen that STAT6 transcriptional activity associated with gene expression for neutrophil migration is effectively prevented by treating 10 and 100 ug/ml of PLAG and 20 nM and 200 nM of STAT6 inhibitors as positive control groups. These data shows that EC-18 mitigates the activity of PKC-ξ/λ and has an effect on the consecutive dephosphorylation of Jak1 within PKC-ξ, JAK1 and STAT6 cascades.

### [Experimental Example 7] Neutrophil adsorption analysis

In order to determine the effect of PLAG on neutrophil adsorption of fibronectin, 24-well tissue culture plates were coated with fibronectin as in previous studies (Wang YH, Wang WY, Liao JF, Chen CF, Hou YC, Liou KT, Chou YC, et al. Prevention of macrophage adhesion molecule-1 (Mac-1)-dependent neutrophil firm adhesion by taxifolin through impairment of protein kinase-dependent NADPH oxidase activation and antagonism of G protein-mediated calcium influx. Biochem Pharmacol 2004; 67:2251-2262.). The plates were incubated with 25 ug/ml of fibronectin at 37°C for 2 hours. The unbounded protein was removed by washing, neutrophils were pre-treated with PLAG 2 hours before the experiment, and neutrophil activity was performed with IL-4 for 4 hours. Non-adsorbed neutrophils were eluted by aspiration and washed twice with PBS. Attached neutrophils were determined by calculating the number of cells at the bottom of each well using a homocytometer. The remaining adsorbed cells were stored in an incubator for 18 hours, at which time the cells were exposed to WST-1 reagent (Roche Applied Science) for spectrometric evaluation.

FIG. 11 is a figure showing the change in expression of IL-8 and VCAM-1 and the effect of decreasing the cell adhesion ability depending on the dose of PLAG when the composition of the present invention was administered. As shown in FIG. 11, RT-PCR data shows that the expression of IL-8 and VCAM-1, which are stimulated and secreted by IL-4 so that HL-60 cells is adsorbed to fibronectin, can be effectively suppressed by PLAG administration (dose-dependent method, etc.) (Fig. 11A). Activity of HL-60 regulated by IL-4 and/or PLAG was calculated using WST-1 assay and cell number. 10 ng/ml of IL-4 is sufficient to stimulate HL-60 cells for adsorption to coated fibronectin, and 100, 10, and 1 ug/mL of PLAG significantly reduced the dose-dependent adsorption of HL-60 (see B and C in FIG. 11).

## Claims

1. A pharmaceutical composition for preventing or treating hepatitis comprising a monoacetyl diacylglycerol compound represented by the following Chemical Formula 1 as an active ingredient: 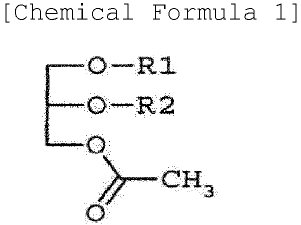 in Chemical Formula 1, R₁ and R₂ are each independently a fatty acid group having 14 to 22 carbon atoms.

2. The pharmaceutical composition for preventing or treating hepatitis of claim 1, wherein the R₁ and R₂ are each independently selected from the group consisting of palmitoyl, oleoyl, linoleoyl, linolenoyl, stearoyl, myristoyl and arachidonoyl.

3. The pharmaceutical composition for preventing or treating hepatitis of claim 1, wherein the monoacetyl diacylglycerol compound is represented by the following Chemical Formula 2.

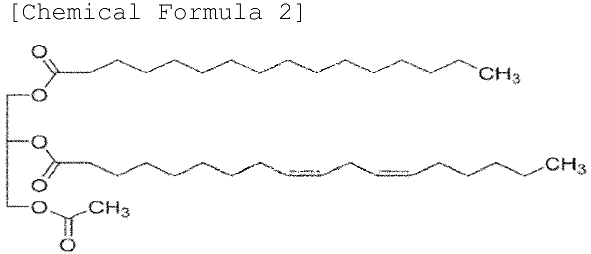

4. The pharmaceutical composition for preventing or treating hepatitis of claim 1, wherein the monoacetyl diacylglycerol compound inhibits the expression and/or activity of IL-4, IL-6 and/or MIP-2.

5. The pharmaceutical composition for preventing or treating hepatitis of claim 4, wherein the MIP-2 is CXCL2 and/or IL-8.

6. The pharmaceutical composition for preventing or treating hepatitis of claim 4, wherein the IL-4 suppresses the phosphorylation of hepatic cytokines, the expression of adsorbed molecules and/or the migration of neutrophils.

7. The pharmaceutical composition for preventing or treating hepatitis of claim 6, wherein the cytokine is selected from the group consisting of PKC-ζ, PKCδ, PKCθ, STAT6 and a mixture thereof.

8. A health functional food composition comprising a monoacetyl diacylglycerol compound represented by the following Chemical Formula 1, as an active ingredient, and is capable of preventing or improving hepatitis: 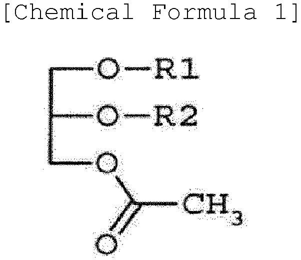 in Chemical Formula 1, R₁ and R₂ are each independently a fatty acid group having 14 to 22 carbon atoms.

9. A method for preventing or treating hepatitis comprising:
administering the composition of any one of claims 1 to 8 to a subject suspected of hepatitis.

10. The method for preventing or treating hepatitis of claim 9, wherein the dose of the monoacetyl diacylglycerol compound contained in the composition is 0.001 to 2000 mg/kg per day.
